# EUROPEAN PATENT SPECIFICATION

(11) **EP 2 349 430 B1**
(45) Date of publication and mention of the grant of the patent: **07.08.2019**
(21) Application number: 09760281.7
(22) Date of filing: 28.10.2009
(51) Int. Cl.: A61M 25/00, A61M 25/09

(54) **CATHETERISATION DEVICE and kit**
KATHETERISIERUNGSVORRICHTUNG und Kit
DISPOSITIF et kit DE CATHÉTÉRISATION

(30) Priority: 31.10.2008 GB 0820052
(43) Date of publication of application: 03.08.2011
(60) Divisional application: 19183481.1
(73) Proprietor: Urethro Tech Ltd., Kingston upon Thames Surrey KT2 5NY (GB)
(72) Inventor: ANDRICH, Daniela, Surrey KT2 5NY (GB)
(74) Representative: Kehoe, Laura Ellen
(86) International application number: PCT/GB2009/051453
(87) International publication number: WO 2010/049735

(56) References cited:
- EP-A- 0 368 330
- FR-A- 2 522 507
- FR-A- 2 633 835
- US-A- 4 811 847
- US-A- 4 938 746
- US-A- 5 391 155
- US-A1- 2004 073 171

## Description

### FIELD

The present invention relates to a device and a related kit for urinary catheterisation.

### BACKGROUND

Catheterisation involves the insertion of a catheter into a body cavity, duct or vessel, thereby allowing drainage or injection of fluids or access by surgical instruments. In the case of urinary catheterisation, a catheter is inserted into a patient's bladder usually for the treatment of urinary incontinence, or to release urine from the bladder when the patient is in acute urinary retention due to, for example, prostate enlargement or post-operatively to monitor urine output when a patient is ill and unable to move. The catheter is normally inserted through the patient's urethra, but if the urethra is blocked or damaged the catheter may need to be inserted into the patient's bladder through the wall of the abdomen instead.

There are two main types of urinary catheterisation. Indwelling catheterisation means that a catheter is left in the body for a period of time. It should be performed by a medical practitioner. Intermittent catheterisation is the temporary placement of the catheter to empty the patient's bladder and may be performed by a medical practitioner or by the patient (in which case it is termed intermittent self-catheterisation).

Catheters for indwelling catheterisation typically have two or three channels and are retained inside the bladder by a balloon located at the tip of the catheter. The balloon is inflated by pumping air, water or saline solution down the narrow balloon channel of the catheter using a syringe. Once inflated, the balloon keeps the catheter securely in the bladder while the primary channel of the catheter allows urinary drainage. An optional third irrigation channel may be used to wash away blood and small clots that may be present after bladder or prostate surgery. This prevents larger clots from forming, which might otherwise block the catheter.

Catheters for intermittent catheterisation do not have a balloon at the tip because they are not intended to be retained in the bladder.

Where a patient has a blocked urine flow due to a narrowing of the urethra, known as a urethral stricture, the stricture will often need to be stretched before a catheter can be inserted. Stretching of the stricture can be achieved using a urethral dilator.

A problem with known types of catheters and dilators, is that correct insertion of the device can be a difficult and potentially harmful procedure due to the natural curvature of the bulbar urethra in men. It can be difficult to advance the catheter/dilator into the part of the urethra known as the prostatic urethra which curves upwards towards the bladder, especially when the prostate is enlarged. The distal tip can cause trauma to the urethral tissue as the user attempts to navigate the catheter along the curved path of the bulbar urethra towards the prostatic urethra. Tissue trauma can lead to the formation of a false passage (i.e. a hole dug into the wall of the urethra), which can make future urethral catheterisation attempts difficult, if not impossible.

If malposition of the catheter is not noticed, inflation of the catheter balloon whilst the tip of the catheter is still in the urethra (a risk associated with known types of indwelling catheters) can seriously damage the urethral tissue, causing pain, bleeding and traumatic rupture. The rupture can heal with a urethral stricture which may require dilatation or surgery at a later date. At worst, if the urethral tissue damage is not noticed in time, subsequent urethral ischaemic necrosis can result in associated life threatening sepsis and loss of urethral and penile tissue.

US5391155 describes a device for insertion into the ureter or renal pelvis and has a ureter tube with an opening of its distal tip, an auxiliary tube and a mandrin. In the insertion position, at least the ureter tube and mandrin are releasably connected. A connecting-piece at the proximal end of the mandrin makes an injection of an X-ray contrast medium into the hollow space inside the mandrin possible where it passes to an outlet opening at the distal end of the mandrin which outlet opening has an entry to the ureter. The mandrin preferably has an inherent curvature in its distal tip and a moveable steering wire can be placed into the lumen of the mandrin.

FR2633835 describes an infusion catheter having a displaceable guide tube which is impermeable and preferably made of PTFE, reinforced by a PTFE coated stainless steel wire. A flexible core of stainless steel can be introduced in the guide tube. There is a need to improve upon current urinary catheterisation devices and kits in order to improve the safety of the procedures, to simplify and speed up insertion and to improve the performance of the devices.

### SUMMARY

In a first aspect, the present invention resides in a device for urinary catheterisation comprising:
a catheter having an elongate body provided with a distal tip, a proximal end, and a drainage channel and a separate guidewire channel extending therethrough;
a guidewire extending through the guidewire channel for guiding the catheter upon insertion; and
a lubrication port at or towards the proximal end of the elongate body, the lubrication port comprising a side arm in fluid communication with the guidewire channel for the introduction of a guidewire lubrication fluid into the guidewire channel;
wherein the drainage channel extends along the centre of the elongate body and the guidewire channel extends parallel to the drainage channel along one side of the elongate body.

Preferably, the guidewire is provided with a stopper in order to prevent the proximal end of the guidewire from passing through the guidewire channel towards the distal tip of the catheter and becoming lost in the urethra or bladder. The stopper may be adapted to receive a syringe for introducing guidewire lubrication fluid into the guidewire channel when the stopper is coupled to the lubrication port. Lubrication of the guidewire is important since it reduces friction between the guidewire and the urethra and also between the guidewire and the catheter.

The stopper may take various forms, for example the stopper may comprise a head portion and a body portion arranged in a T-shaped configuration and having a hollow core extending therethrough, with the body portion capable of being received in the lubrication port and the head portion capable of receiving the syringe. Alternatively, the stopper may comprise only a head portion having a hollow core extending therethrough, the head portion capable of receiving the syringe. In each configuration of stopper the head portion may suitably be larger than the opening of the lubrication port. The stopper may be releasably connected to the lubrication port, preferably by a screw means, a push-fit means or a twist-lock means, though other mechanisms such as a snap-lock or a Luer Lock may equally be used.

Moreover, the stopper may be provided with an anchor for connecting the proximal end of the guidewire to the stopper. The anchor may be suitably configured so as to allow the guidewire lubrication fluid to flow around the anchor as it passes through the stopper and into the guidewire channel. Preferably, the anchor comprises a band, which traverses the width of the hollow core of the stopper, and end members at either end of the band, which are embedded in either side of the body of the stopper so as to secure the anchor in the stopper. More preferably, the anchor may be I-shaped. In a preferred arrangement, the proximal end of the guidewire is connected to the band of the anchor. Suitable means of connection include welding when, for example, the anchor is formed of metal and an epoxy resin when, for example, the anchor comprises a rigid polymeric material.

The catheter further includes a drainage channel separate from the guidewire channel for the drainage of urine. The drainage channel extends along the centre of the elongate body and the guidewire channel extends parallel to the drainage channel along one side of the elongate body. Optionally, the guidewire channel may also act as an irrigation channel for the introduction of sterile irrigating solution into the bladder once the guidewire has been removed.

The lubrication port comprises a side arm in fluid communication with the guidewire channel adjacent to the proximal end of the catheter. The lubrication port may be provided with a closure member for preventing drainage of urine through the lubrication port when the guidewire has been removed. Preferably, the closure member is a one-way valve.

Advantageously, the guidewire extends beyond the distal tip of the catheter when the stopper is coupled to the lubrication port. Preferably, the guidewire comprises a hydrophilic polymeric material.

The distal tip of the catheter may be open-ended so as to allow for drainage of urine from the bladder. Beneficially, this feature may also allow for the catheter to be used with known guidewires for the purpose of changing a long-term urethral catheter, particularly a suprapubic catheter. This makes the catheter changing procedure much safer and easier because the tract of the supra-pubic catheter cannot be lost. This is a frequent issue when inadequately trained clinical practitioners change suprapubic catheters.

Preferably, the elongate body of the catheter may be provided with at least one further drainage hole adjacent to the distal tip of the catheter so as to increase drainage efficiency. More preferably, the catheter may be provided with two drainage holes, which are located adjacent to the distal tip, one on either side of the catheter, so as to further increase drainage efficiency. Moreover, the presence of three drainage holes means that even if one or two of the holes become blocked, for example with a blood clot, drainage of urine can continue to take place through the other drainage hole.

The distal tip of the catheter is preferably formed at an oblique angle so as to help reduce tissue trauma as the catheter passes through the urethra. The oblique angle also provides a leading edge which helps the catheter to follow the guidewire in the bulbar and prostatic urethra.

The catheter may further comprise an inflatable balloon located adjacent to the distal tip of the catheter for retaining the catheter inside the bladder. In order to inflate the balloon, the catheter may include an inflation channel for introducing inflation fluid into the balloon.

The catheter may typically be made from a flexible, resilient and biocompatible polymeric material, such as polytetrafluoroethene (PTFE), polyethylene, polypropylene, polyurethane, polyvinyl chloride (PVC), latex or silicone. The inflatable balloon can be made from the same material as the catheter and may comprise a fine distensible additional layer of polymeric material attached to the elongate shaft.

To promote ease of insertion, withdrawal and positioning of the catheter, the catheter may optionally be coated with a hydrophilic lubricious polymer, such as polyurea, polyurethane, polyurethaneurea, polyglycols, polyvinyl pyrrolidone (PVP) or carboxylic acids, esters, salts and amides of poly(meth)acrylic acid, which becomes slippery in the presence of an aqueous fluid. Additionally or alternatively, a tube of lubricant such as Lidocaine/Aquagel or Aquagel alone can be injected into the urethra. To reduce and delay the occurrence of an infection, the catheter may also be coated with an anti-infective material such as a silver alloy or an antibiotic.

The size of the catheter may typically be between 10 French and 26 French in diameter and between 20 cm and 50 cm in length. A longer length catheter is usually required for males since the male urinary tract is longer than the female urinary tract.

Described herein, but not forming part of the invention, is a method of urinary catheterisation, comprising:
lubricating a guidewire located in a channel extending through a catheter, the catheter having an elongate body provided with a distal tip, a proximal end and one or more channels extending therethrough, and the guidewire having a distal tip and a proximal end;
inserting the distal tip of the guidewire into the urethra and navigating the guidewire along the urethra and into the bladder;
advancing the catheter over the guidewire until the distal tip of the catheter is located in the bladder; and
removing the guidewire from the urethra and the catheter.

Also described herein, but not forming part of the invention, is a device for urethral stricture dilatation comprising:
a dilator having an elongate body provided with a distal end tapered towards a distal tip, a proximal end and a guidewire channel extending therethrough;
a guidewire extending through the guidewire channel for guiding the dilator upon insertion; and
a lubrication port at the proximal end of the elongate body for introduction of a guidewire lubrication fluid into the guidewire channel.

The distal end of the dilator may taper along a length of between 3 cm and 4 cm towards the distal tip. Furthermore, the distal end of the dilator may be angled relative to the elongate body. Preferably, the distal end may be angled between 30° and 45° relative to the elongate body. Conveniently, the size of the dilator may range between 2-14 French and 2-24 French.

The dilator may typically be made from a resilient and biocompatible polymeric material so that it can push through the scar tissue of a urethral stricture. The tapered distal end of the dilator may be made from a different material to the remainder of the elongate body. For example, the tapered distal end may be made from a material which provides it with greater flexibility, reduced flexibility, greater slipperiness or reduced slipperiness compared to the remainder of the elongate body. Alternatively, or in addition thereto, the tapered distal end may be made from a material whose physical properties vary depending on the surrounding temperature. For example, the material may be one that distends (swells) upon contact with the patient's body but contracts upon contact with cold water. To promote ease of insertion, withdrawal and positioning of the dilator, the dilator may optionally be coated with a hydrophilic lubricious polymer, such as polyurea, polyurethane, polyurethaneurea, polyglycols, polyvinyl pyrrolidone (PVP) or carboxylic acids, esters, salts and amides of poly(meth)acrylic acid, which becomes slippery in the presence of an aqueous fluid. Additionally or alternatively, lubricant such as Lidocaine/Aquagel or Aquagel alone can be injected into the urethra. To reduce and delay the occurrence of an infection, the dilator may also be coated with an anti-infective material such as a silver alloy or an antibiotic.

Further described herein, but not forming part of the invention, is a method of urethral stricture dilatation, comprising:
lubricating a guidewire located in a guidewire channel extending through a dilator, the dilator having an elongate body provided with a distal end, tapered towards a distal tip, and a proximal end, and the guidewire having a distal tip and a proximal end;
inserting the distal tip of the guidewire into the urethra and navigating the guidewire along the urethra and into the bladder;
advancing the dilator over the guidewire until the distal tip of the dilator is located in the bladder; and
removing the guidewire and the dilator from the urethra.

Further described herein, but not forming part of the invention in itself, is a guidewire for use in a medical device, wherein the guidewire is provided with a stopper for preventing loss of the guidewire. Preferably, the stopper is adapted to receive a syringe for introducing guidewire lubrication fluid onto the guidewire.

Advantageously, the catheter of the present invention may be used with known guidewires, for example, those comprising a core wire coated with hydrophilic polymer.

In a second aspect, the present invention resides in a urinary catheterisation kit comprising:
a catheter having an elongate body provided with a distal tip, a proximal end, and a drainage channel and a separate guidewire channel extending therethrough, wherein the drainage channel extends along the centre of the elongate body and the guidewire channel extends parallel to the drainage channel along one side of the elongate body;
a guidewire extending through the guidewire channel for guiding the catheter upon insertion;
a lubrication port at or towards the proximal end of the elongate body, the lubrication port comprising a side arm in fluid communication with the guidewire channel for introduction of a guidewire lubrication fluid into the guidewire channel; and
a sterile package in which the catheter and guidewire are supplied for use, the package adapted to be opened to allow injection of guidewire lubrication fluid through the lubrication port to lubricate the guidewire in the guidewire channel and that portion of the guidewire extending beyond the distal tip of the catheter.

The kit may further comprise an external sterile package for housing the sterile package containing the catheter and guidewire, wherein the external sterile package additionally houses at least one syringe. Preferably, the at least one syringe is pre-filled with guidewire lubrication fluid.

Additionally described herein, but not forming part of the invention, is a urethral stricture dilatation kit comprising:
a dilator having an elongate body provided with a distal end tapered towards a distal tip, a proximal end and a guidewire channel extending therethrough;
a guidewire extending through the guidewire channel for guiding the dilator upon insertion;
a lubrication port at the proximal end of the elongate body for introduction of a guidewire lubrication fluid into the guidewire channel; and
a sterile package in which the dilator and guidewire are supplied for use, the package adapted to be opened to allow injection of guidewire lubrication fluid through the lubrication port to lubricate the guidewire in the guidewire channel and that portion of the guidewire extending beyond the distal tip of the dilator.

The kit may further comprise an external sterile package for housing the sterile package containing the dilator and guidewire, wherein the external sterile package additionally houses a syringe. Preferably, the syringe is pre-filled with guidewire lubrication fluid.

The present invention has the advantage of simplifying and improving the safety of the catheterisation procedure. The integration of the guidewire into the catheter facilitates placement of the catheter in the urethra and provides a convenient and safe product for use by a medical/clinical practitioner or patient.

The guidewire of the device/kit of the present invention is especially advantageous because the stopper prevents loss of the proximal end of the guidewire along the guidewire channel into the urethra or bladder. Moreover, when the stopper is capable of receiving a syringe filled with lubrication fluid, it enables the hydrophilic guidewire to be lubricated whilst it is located within the catheter/dilator. This helps to maintain the sterility of the device whilst it is prepared for use and makes preparation more convenient for the user. Also, the fact that the device is still retained inside its internal wrapping at the time of lubrication ensures that the guidewire is fully lubricated along its entire length.

A number of definitions are provided that will assist in the understanding of the invention.

The term 'catheter' as used herein denotes a hollow tube used to drain fluid from a body cavity, duct or vessel, or to distend a body passage, and the term 'catheterisation' denotes the operation of introducing a catheter into the body.

The term 'dilator' as used herein denotes an instrument for dilating or distending a body cavity, duct, vessel, canal or orifices, and the term 'dilatation' as used herein denotes the operation of introducing a dilator into the body.

### BRIEF DESCRIPTION OF THE DRAWINGS

In order that the invention may be more readily understood, reference will now be made, by way of example, to the accompanying drawings in which:
Figure 1 is a device for urinary catheterisation in a first embodiment of the present invention: (a) is a side view of an indwelling catheter having a drainage channel, a balloon inflation channel and a guidewire channel with guidewire in-situ; (b) is a cross-sectional view of the catheter taken along the line II-II; (c) is a cross-sectional view of the catheter taken along the line III-III.
Figure 2 is a guidewire having a stopper for use in all embodiments of the present invention: (a) is a perspective view of a guidewire having a T-shaped stopper; (b) is a perspective view of a guidewire having a cylindrical stopper; (c) is an end view of the stopper as shown in (a) and (b).
Figure 3 is a side view of a device for urinary catheterisation in a second embodiment of the present invention.
Figure 4 is a side view of a device for urethral stricture dilatation as described herein but not forming part of the present invention.
Figure 5 is a top view of the sterile packaging for a device of all embodiments of the present invention.

### DETAILED DESCRIPTION

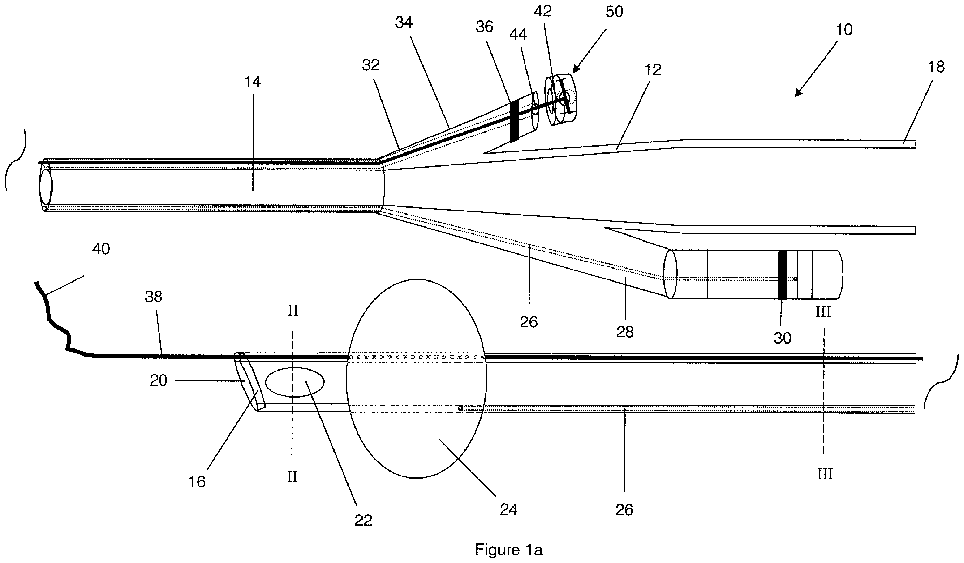

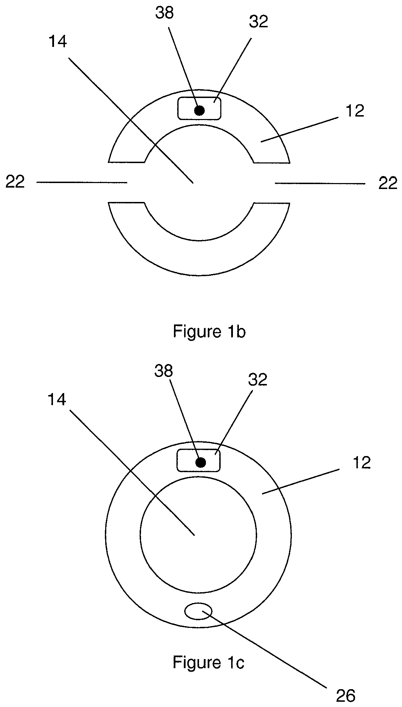

A first embodiment of the invention is shown in Figure 1. The device comprises a catheter 10 having a flexible elongate shaft 12. The elongate shaft 12 defines a drainage channel 14 which extends along the length of the catheter 10 from the distal tip 16 of the catheter 10 to the proximal end 18 of the catheter 10. The proximal end 18 of the catheter 10 is capable of being connected to a fluid collector (not shown).

The distal tip 16 of the catheter is open-ended and formed at an oblique angle. The open end of the distal tip 16 defines a drainage hole 20, which allows fluid to enter the drainage channel 14. Two further drainage holes 22 are located adjacent to the distal tip 16, one on each side of the elongate shaft 12, to increase drainage efficiency of the device.

An inflatable balloon 24 is located adjacent to the distal tip 16 of the catheter 10 and encircles the elongate shaft 12. Typically, the balloon 24 has a maximum volume of between 10 ml and 30 ml and is spherical in shape, although various shapes may be used. The wall of the elongate shaft 12 houses an inflation channel 26, which extends along the length of the catheter 10 from the inflatable balloon 24 to an inflation channel side arm 28 positioned adjacent to the proximal end 18 of the catheter 10. The inflation channel 26 is cylindrical and typically has a diameter between 0.1 mm and 0.3 mm. The inflation channel side arm 28 houses a two-way valve 30 and is capable of receiving a syringe. The balloon 24 is inflated by inserting a syringe (not shown) filled with air, water or saline solution into the inflation channel side arm 28 and injecting the contents of the syringe into the inflation channel side arm 28, through the valve 30, and along the inflation channel 26 until the balloon 24 is sufficiently expanded. The valve 30 prevents the contents of the inflated balloon 24 from leaking out of the inflation channel side arm 28 until deflation is required. To deflate the balloon 24, an empty syringe is connected to the inflation channel side arm 28 and the contents of the balloon 24 are removed by suction.

The wall of the elongate shaft 12 further comprises a guidewire channel 32, which extends along the length of the catheter 10 from the distal tip 16 of the catheter 10 to a guidewire channel side arm 34 positioned adjacent to the proximal end 18 of the catheter 10 on the opposite side of the shaft to the inflation channel side arm 28. The guidewire channel 32 is oblong in cross-section although other shapes may be used, for example, oval, circular, or square in cross-section, and typically has dimensions of about 1-2 mm x 1mm. Both the guidewire channel 32 and the inflation channel 26 run parallel to the drainage channel 14 and are independent from the drainage channel 14. The guidewire channel side arm 34 has a one-way valve 36 to prevent urine from draining along the guidewire channel 32 and out of the opening 44 of the guidewire channel 32 when the guidewire 38 has been removed from the device. Alternative means of closing the guidewire channel 32 may be used.

A flexible guidewire 38 is located in the guidewire channel 32 and serves to guide the catheter 10 into the correct position upon insertion. The guidewire 38 is typically between 0.8 mm and 0.9 mm in diameter and between 100 cm and 180 cm in length, and extends from the proximal end of the guidewire channel side arm 34, along the full length of the guidewire channel 32 and beyond the distal tip 16 of the catheter 10.

The guidewire 38 comprises a core wire, typically made of stainless steel, platinum, shape memory alloys such as Nitinol, or other metal, which provides a degree of stiffness to the guidewire 38. The core wire extends almost the entire length of the guidewire 38, terminating shortly before the guidewire distal tip 40, and may taper in diameter towards the distal tip 40, thereby increasing the flexibility of the guidewire 38 towards the distal tip 40. The highly flexible distal tip 40, without the core wire, is typically between 3 cm and 9 cm in length. The flexibility of the distal tip 40 reduces tissue damage and degradation as the guidewire 38 enters the body and enables the user to safely and easily manoeuvre and navigate the guidewire 38 along the urinary tract towards the bladder.

The core wire of the guidewire 38 is coated with a hydrophilic polymer, such as polyurea, polyurethane, polyurethaneurea, polyglycols, polyvinyl pyrrolidone (PVP) or carboxylic acids, esters, salts and amides of poly(meth)acrylic acid. The hydrophilic coating becomes slippery in the presence of an aqueous fluid, such as water, and provides surface lubricity to the guidewire 38. This surface lubricity reduces friction between the guidewire 38 and body tissue as the guidewire 38 passes through the urinary tract and also reduces friction between the guidewire 38 and the guidewire channel 32 as the catheter 10 is passed over the guidewire 38.

The hydrophilic coating can comprise a simple coating of one polymer, a blending/complexing of two or more hydrophilic polymers, an interpenetrating network of polymers or one or more chemically reactive hydrophilic polymers.

A stopper 50 located at the proximal end 42 of the guidewire 38 prevents the guidewire 38 from disappearing along the guidewire channel 32 towards the distal tip 16 of the catheter 10 and becoming lost in the urethra or bladder.

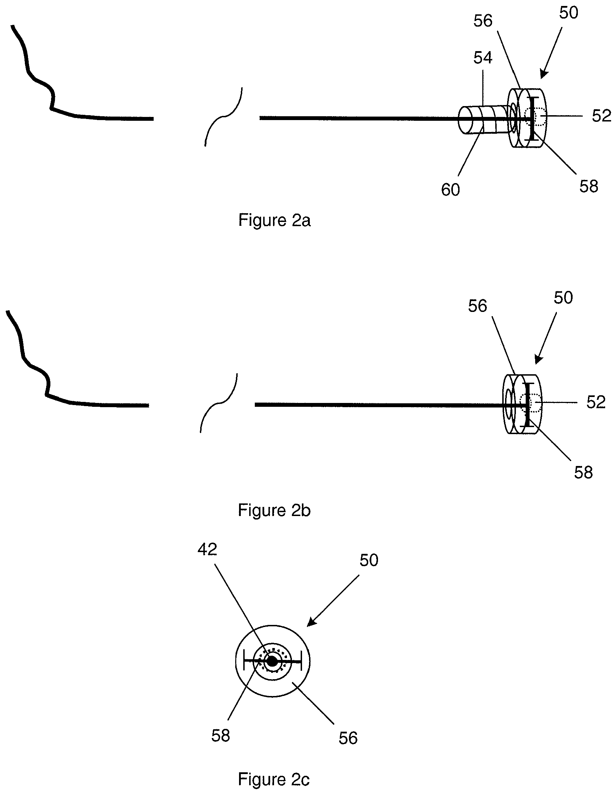

Figures 2(a) and 2(b) show two different stopper designs in more detail. Figure 2(a) shows a stopper 50 having a T-shaped configuration and comprising a hollow core 52 which extends through the body 54 and the head 56 of the stopper 50. The head 56 of the stopper 50 contains a narrow metal anchor 58 which traverses the hollow core 52 and is embedded in either side of the head 56 of the stopper 50. The proximal end 42 of the guidewire 38 is welded to the metal anchor 58, thereby securely connecting the guidewire 38 to the stopper 50.

The head 56 of the stopper 50 is capable of receiving a syringe (not shown), with the nose of the syringe fitting into the hollow core 52 of the stopper 50. To lubricate the guidewire 38, a syringe filled with aqueous fluid, such as water, is inserted into the head 56 of the stopper 50 and the fluid is injected into the stopper 50, through the guidewire channel side arm 34 and along the guidewire channel 32 towards the distal tip 16 of the catheter 10. The narrow width of the metal anchor 58 in the head 56 of the stopper 50 means that fluid can easily move past the anchor 58 as it passes through the stopper 50.

The body 54 of the stopper 50 is sized so as to be able to fit into the guidewire channel side arm 34, whilst the head 56 of the stopper 50 is larger than the opening 44 of the guidewire channel 32 and abuts the opening 44 so as to prevent the stopper 50 from passing through the guidewire channel 32 towards the distal tip 16 of the catheter 10. The stopper 50 is releasably connected to the guidewire channel side arm 34 by means of a screw thread 60 on the outer surface of the body 54 of the stopper 50 which interacts with a complimentary screw thread on the inner surface of the guidewire channel 32 to provide a secure connection between the guidewire 38 and the catheter 10 prior to and during use of the guidewire 38.

An alternative configuration of stopper is shown in Figure 2(b). In this instance, the stopper 50 comprises only a head 56 having a hollow core 52 into which the nose of a syringe fits. The head 56 of the stopper 50 is connected to the proximal end 42 of the guidewire 38 by means of an anchor 58, as previously described, and abuts the opening 44 of the guidewire channel 32 to prevent the stopper 50 from passing through the guidewire channel 32 towards the distal tip 16 of the catheter 10. To enable a secure connection between the stopper 50 and the catheter 10, the head 56 of the stopper 50 can be releasably coupled to the guidewire channel side arm 34 as previously described with reference to Figure 2(a). Figure 2(c) shows an end view of the stopper 50 of Figure 2(a) and 2(b) and the configuration of the anchor 58 traversing the hollow width 52 of the stopper 50.

In an exemplary use, the device of a first embodiment of the invention, as shown in Figure 1, is inserted into the patient's urinary tract and retained in the bladder to drain away urine in the following manner.

Firstly, the full length of guidewire 38 is lubricated by inserting a syringe filled with water into the stopper 50 located at the proximal end 42 of the guidewire 38 and injecting the contents of the syringe into the stopper 50, along the guidewire channel side arm 34 and along the full length of the guidewire channel 32. Upon contact with the water, the hydrophilic coating of the guidewire 38 swells and becomes slippery and provides surface lubricity to the guidewire 38. The surface lubricity helps to reduce friction between the guidewire 38, the guidewire channel 32 and body tissue as the guidewire 38 passes through the urinary tract. Once the guidewire 38 is fully lubricated, it is then ready to be inserted into the urethra of the patient.

A lubricant is inserted into the urethra of the patient, then the tip 40 of the guidewire 38 is inserted into the urethra and the guidewire 38 is navigated along the path of the urethra towards the bladder. The high flexibility of the guidewire tip 40 reduces tissue damage and degradation as the guidewire 38 moves along the urinary tract and helps the user to circumvent any obstructions in the urethra. By inserting the entire length of the guidewire 38 the user can be sure that the guidewire 38 has reached the bladder. The presence of the stopper 50 prevents the guidewire 38 from disappearing along the guidewire channel 32.

Once the guidewire 38 is fully inserted into the bladder, the catheter 10 is ready to be advanced over the guidewire 38. The surface lubricity of the guidewire 38 , combined with the optional surface lubricity of the catheter 10, reduces the frictional resistance between the guidewire 38 and the guidewire channel 32 as the catheter 10 slides over the guidewire 38. The guidewire 38 defines the path along which the catheter 10 travels and makes catheter insertion much safer and more reliable, thereby causing minimal discomfort to the patient.

The catheter 10 is in its fully inserted position when the distal tip 16 of the catheter 10 reaches the bladder and the balloon 24 is beyond the bladder neck. The user can assess whether or not the end of the catheter 10 is correctly positioned in the bladder by observing the drainage of urine through the drainage channel 14 of the catheter 10. If no urine drainage is observed, the distal tip 16 of the catheter 10 is still in the urethra and the catheter 10 will need to be advanced further along the guidewire 38. Once urine drainage is observed, the user can be sure that the catheter 10 is in the correct position and the guidewire 38 can safely be removed from the bladder and the catheter 10. The catheter 10 is then connected at its proximal end 18 to a fluid collector (not shown).

To retain the catheter 10 in the bladder, the balloon 24 is inflated by inserting a syringe filled with water or saline solution into the inflation channel side arm 28 and injecting the contents of the syringe into the inflation channel side arm 28 and along the inflation channel 26 until the balloon 24 is filled. The catheter 10 remains in place until the patient is able to void again after surgical treatment, until measurement of urine output is no longer required or until the catheter 10 needs to be replaced. To remove the catheter 10, the balloon 24 is deflated by connecting an empty syringe to the inflation channel side arm 28 and sucking the water or saline solution out of the balloon 24, and then the catheter 10 is carefully removed.

The guidewire channel 32 can optionally be used as an irrigation channel, once the catheter 10 has been fully inserted and the guidewire 38 removed, to flush sterile irrigating solution into the bladder. In this instance, an irrigation fluid connector (not shown) is attached to the guidewire channel side arm 34 using a releasable connection mechanism such as a screw fit, a push-fit, a snap-lock, a twist-lock or a Luer Lock. Once the bladder has been suitably irrigated and cleared of blood clots etc., the irrigation fluid connector is removed.

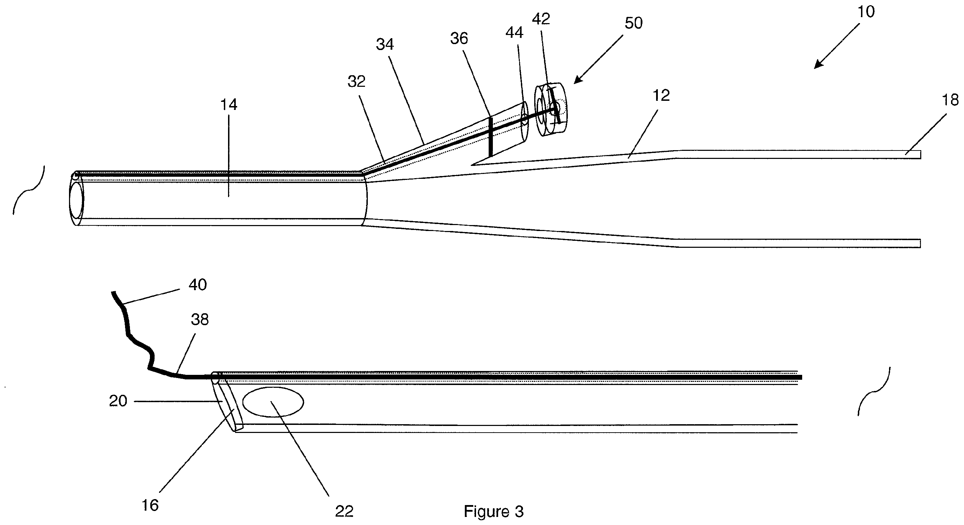

A second embodiment of the invention is shown in Figure 3. This device is intended for patients who require intermittent catheterisation in order to empty their bladder because of bladder failure or after bladder reconstruction. The device does not need to be retained in the bladder so it does not have an inflatable balloon but it is otherwise identical to the first embodiment of the invention. In this instance, the catheter 10 is inserted into the patient's bladder and retained there only until complete drainage of urine from the bladder has been observed.

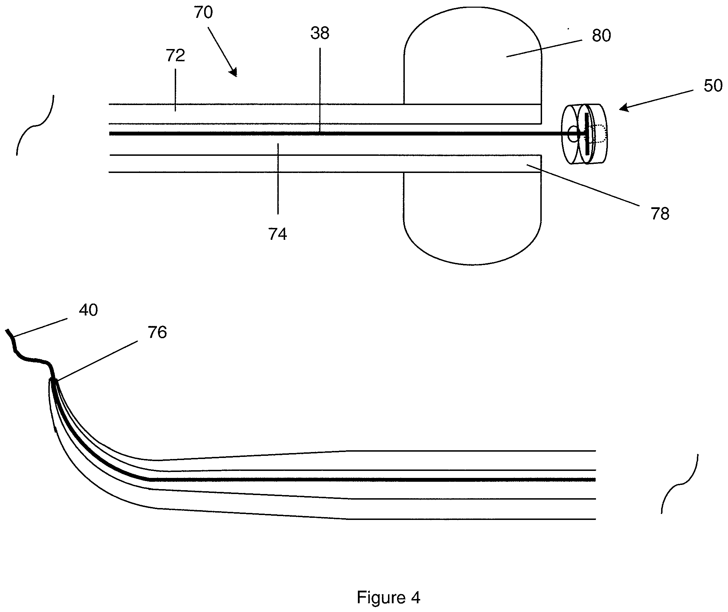

An exemplary device, not forming part of the invention, is shown in Figure 4. This device is for dilating urethral strictures and comprises a dilator 70 having an elongate shaft 72. The elongate shaft 72 defines a guidewire channel 74 which extends along the length of the dilator 70 from the distal tip 76 of the dilator 70 to the proximal end 78 of the dilator 70. The guidewire channel 74 is cylindrical and typically has a diameter between 0.8 mm and 1.0 mm, although it tapers at the distal end 76 of the elongate shaft 72 towards the distal tip 76. Both the distal tip 76 and the proximal end 78 of the guidewire channel 74 are open-ended and the proximal end 78 is provided with a handle 80 for ease of use.

A flexible guidewire 38 is located in the guidewire channel 74 and serves to guide the dilator 70 into the correct position upon insertion. The guidewire 38 and stopper 50 of this example is identical to that used in the first and second embodiment of the invention. As per the previous embodiments, the stopper 50 located at the proximal end 42 of the guidewire 38 prevents the guidewire 38 from disappearing along the guidewire channel 74, except in this example there is no guidewire channel side arm so the stopper 50 interacts with the proximal end 78 of the dilator 70 rather than the proximal end of the guidewire channel side arm.

The size of the dilator 70 is typically between 2-14 French and 2-24 French in diameter and between 30 cm and 40 cm in length. The distal end 77 of the dilator 70 is curved at an angle of approximately 30-45° to help follow the natural curve of the urethra, although straight tips may also be used. Moreover, the distal end 77 is tapered over a length of approximately 4 cm towards the distal tip 76 from a diameter of between 14 French and 24 French at the widest point of the elongate shaft 72 down to 2 French at the distal tip 76. The dilator 70 also has circumferential markings (not shown) every 10 cm along the length of the elongate shaft 72 in order to assist the medical/clinical practitioner in assessing the length of dilator 70 that has been inserted into the urethra. The close fit of the distal tip 76 around the guidewire 38 helps to prevent urethral tissue from becoming caught between the distal tip 76 and the guidewire 38 and therefore reduces the likelihood of tissue damage. Furthermore, the tapered distal end 77 and the narrow diameter of the distal tip 76 causes the distal end 77 of the dilator to be more flexible than the remainder of the wider elongate shaft 72.

The relatively long tapered distal end 77 of the dilator 70 compared to known dilators allows for a large increase in diameter in a single dilator. This enables the user to dilate the stricture up to the maximum width required using a single dilator rather than having to use multiple dilators each having a slightly increased diameter (e.g. 6/10F, 8/12F, 12/14F, 14/18F, etc. whereby the first number denotes the calibre of the tip and the second number denotes the calibre of the shaft). Using one dilator safely over a guidewire speeds up the dilatation procedure and reduces the number of dilators normally used to treat a urethral stricture and hence the amount of waste material produced.

In an exemplary use, the device as shown in Figure 4 is inserted into the patient's urinary tract to dilate a urethral stricture in the following manner.

Firstly, the full length of the guidewire 38 is lubricated and fully inserted into the urethra as per the first and second embodiment of the invention. The highly flexible tip 40 of the guidewire 38 helps the user to find the lumen of the urethral stricture. Once the guidewire 38 has been advanced into the bladder, the dilator 70 is ready to be advanced over the guidewire 38. The dilator 70 is inserted until the distal tip 76 reaches the bladder thereby stretching the stricture to the calibre of the dilator shaft. The dilator 70 and the guidewire 38 are then removed from the urethra.

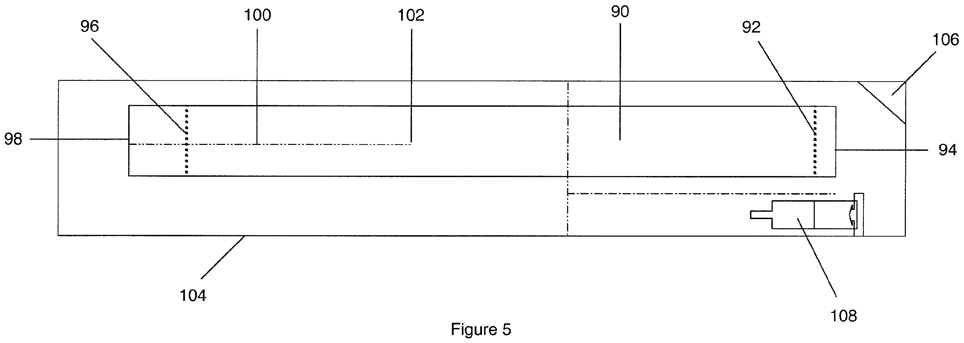

Figure 5 shows how the device of the invention is packaged in a sterile environment ready for use. This packaging can be used for all the embodiments of the invention, i.e. an indwelling catheter (Figure 1), an intermittent catheter (Figure 3) or an exemplary dilator (Figure 4), having an integral guidewire 38. The packaging enables the device to be prepared for use in a convenient and sterile manner.

The device of the invention (not shown) is contained within a sterile internal wrapping 90, which is typically made from plastic. The internal wrapping 90 has a top side and a bottom side (not shown) and is substantially rectangular in shape for ease of manufacture, though other shapes are equally suitable. A first perforation 92 is located at the proximal end 94 of the internal wrapping 90, which is where the proximal end of the device is situated. A second perforation 96 is located at the distal end 98 of the internal wrapping 90, which is where the distal tip of the device is situated. Both the first and second perforations extend across the full width of the top and bottom side of the internal wrapping 90, enabling the internal wrapping 90 to be completely torn at these positions. A third perforation 100 extends along the top side of the internal wrapping 90 from the distal end 98 to approximately the mid-point 102 of the wrapping 90.

The internal wrapping 90 is housed within a sterile external wrapping 104, which is typically made of plastic or paper. The external wrapping 104 has a top side, a bottom side (not shown) and is substantially rectangular in shape, and comprises a peelable corner 106 at its proximal end. Additionally housed within the external wrapping 104 is a syringe 108 pre-filled with water for lubricating the guidewire 38. Where the device comprises an indwelling catheter 10 with an integral guidewire 38, the external wrapping 104 houses two syringes filled with water; one for lubricating the guidewire and one for inflating the balloon. Alternatively, the syringes within the external wrapping 104 may be empty and can be filled by the user when the device is ready to be used.

The device of the invention is prepared for use in the following manner. First, the external wrapping 104 is opened by pulling at the peelable corner 106 at the proximal end of the wrapping. The contents of the external wrapping 104, which include the internal wrapping 90 containing the device and at least one syringe 108 filled with water, are then transferred onto a sterile surface, such as a table or the internal sterile side of the external wrapping.

Next, the first perforation at the proximal end 92 of the internal wrapping 92 is torn to expose the proximal end of the device. The syringe 108 filled with water is then inserted into the stopper 50 located at the proximal end of the guidewire 38 of the device and the water is injected through the stopper 50 in order to lubricate the guidewire 38. The entire length of the guidewire 38 becomes lubricated by virtue of the fact that it is curled up within the internal wrapping 90 and becomes immersed in the water.

Once the guidewire 38 has been fully lubricated, the second and third perforations 96, 100 of the internal wrapping 90 are torn to expose the distal tip of the device. The internal wrapping 90 can then be discarded or it can be kept in position until after the guidewire 38 has been inserted in order to further protect the catheter/exemplary dilator.

Although particular embodiments of the invention have been disclosed herein in detail, this has been done by way of example and for the purposes of illustration only.

## Claims

1. A device for urinary catheterisation comprising:
a catheter 10 having an elongate body 12 provided with a distal tip 16, a proximal end 18, and a drainage channel 14 and a separate guidewire channel 32 extending therethrough;
a guidewire 38 extending through the guidewire channel for guiding the catheter upon insertion; and
a lubrication port 34 at or towards the proximal end of the elongate body, the lubrication port comprising a side arm in fluid communication with the guidewire channel for the introduction of a guidewire lubrication fluid into the guidewire channel;
wherein the drainage channel extends along the centre of the elongate body and the guidewire channel extends parallel to the drainage channel along one side of the elongate body.

2. The device according to claim 1, wherein the guidewire is provided with a stopper 50 for preventing the proximal end of the guidewire from passing through the guidewire channel towards the distal tip of the catheter.

3. The device according to claim 2, wherein the stopper is adapted to receive a syringe for introducing guidewire lubrication fluid into the guidewire channel when the stopper is coupled to the lubrication port.

4. The device according to claim 2 or claim 3, wherein the stopper is capable of being releasably connected to the lubrication port.

5. The device according to any of claims 2 to 4, wherein the stopper is provided with an anchor 58 for connecting the proximal end of the guidewire to the stopper, the anchor being configured so as to allow the guidewire lubrication fluid to flow around the anchor as it passes through the stopper and into the guidewire channel.

6. The device according to claim 5, wherein the anchor comprises a band, which traverses the width of a hollow core of the stopper, and end members at either end of the band, which are embedded in either side of the stopper to secure the anchor in the stopper.

7. The device according to any previous claim, wherein the lubrication port is provided with a closure member for preventing drainage of urine through the lubrication port when the guidewire has been removed.

8. The device according to claim 7, wherein the closure member is a one-way valve 36.

9. The device according to any previous claim, wherein the guidewire channel also acts as an irrigation channel for the introduction of sterile irrigating solution into the bladder once the guidewire has been removed.

10. The device according to any previous claim, wherein the guidewire comprises a hydrophilic polymeric material.

11. The device according to any previous claim, wherein the distal tip of the catheter is open-ended for drainage of urine from the bladder and is formed at an oblique angle.

12. The device according to any previous claim, wherein the catheter further comprises an inflation channel for introducing inflation fluid into an inflatable balloon located adjacent to the distal tip of the catheter for retaining the catheter inside the bladder.

13. A urinary catheterisation kit comprising:
a catheter 10 having an elongate body 12 provided with a distal tip 16, a proximal end 18, and a drainage channel 14 and a separate guidewire channel 32 extending therethrough, wherein the drainage channel extends along the centre of the elongate body and the guidewire channel extends parallel to the drainage channel along one side of the elongate body;
a guidewire 38 extending through the guidewire channel for guiding the catheter upon insertion;
a lubrication port 34 at or towards the proximal end of the elongate body, the lubrication port comprising a side arm in fluid communication with the guidewire channel for introduction of a guidewire lubrication fluid into the guidewire channel; and
a sterile package 90 in which the catheter and guidewire are supplied for use, the package adapted to be opened to allow injection of guidewire lubrication fluid through the lubrication port to lubricate the guidewire in the guidewire channel and that portion of the guidewire extending beyond the distal tip of the catheter.

14. The kit according to claim 13 further comprising an external sterile package 104 for housing the sterile package containing the catheter and guidewire, wherein the external sterile package additionally houses at least one syringe 108, and wherein the at least one syringe is pre-filled with guidewire lubrication fluid.

## Patentansprüche

1. Vorrichtung zur Harnkatheterisierung, Folgendes umfassend:
einen Katheter 10 mit einem länglichen Körper 12, der mit einer distalen Spitze 16, einem proximalen Ende 18 und einem Ableitungskanal 14 und einem separaten Führungsdrahtkanal 32, die sich dadurch erstrecken, versehen ist;
einen Führungsdraht 38, der sich durch den Führungsdrahtkanal erstreckt, zum Führen des Katheters beim Einlegen; und
einen Gleitmittelzufuhranschluss 34 an oder nahe dem proximalen Ende des länglichen Körpers, wobei der Gleitmittelzufuhranschluss einen in Fluidverbindung mit dem Führungsdrahtkanal stehenden Seitenarm für die Einbringung eines Führungsdraht-Gleitfluids in den Führungsdrahtkanal umfasst;
wobei der Ableitungskanal sich entlang der Mitte des länglichen Körpers erstreckt und der Führungsdrahtkanal sich parallel zu dem Ableitungskanal entlang einer Seite des länglichen Körpers erstreckt.

2. Vorrichtung nach Anspruch 1, wobei der Führungsdraht mit einem Stopper 50 versehen ist, um zu verhindern, dass das proximale Ende des Führungsdrahtes den Führungsdrahtkanal in Richtung der distalen Spitze des Katheters passiert.

3. Vorrichtung nach Anspruch 2, wobei der Stopper dazu angepasst ist, eine Spritze zum Einbringen von Führungsdraht-Gleitfluid in den Führungsdrahtkanal aufzunehmen, wenn der Stopper mit dem Gleitmittelzufuhranschluss gekoppelt ist.

4. Vorrichtung nach Anspruch 2 oder Anspruch 3, wobei der Stopper lösbar mit dem Gleitmittelzufuhranschluss verbindbar ist.

5. Vorrichtung nach einem der Ansprüche 2 bis 4, wobei der Stopper mit einem Anker 58 zum Verbinden des proximalen Endes des Führungsdrahtes mit dem Stopper versehen ist, wobei der Anker so ausgestaltet ist, dass das Führungsdraht-Gleitfluid um den Anker herum fließen kann, wenn es den Stopper passiert und in den Führungsdrahtkanal eintritt.

6. Vorrichtung nach Anspruch 5, wobei der Anker ein Band, das die Breite eines hohlen Kerns des Stoppers überspannt, und Endelemente an beiden Enden des Bandes, die in beiden Seiten des Stoppers eingebettet sind, um den Anker in dem Stopper zu sichern, umfasst.

7. Vorrichtung nach einem vorangehenden Anspruch, wobei der Gleitmittelzufuhranschluss mit einem Verschlusselement zum Verhindern eines Abfließens von Harn über den Gleitmittelzufuhranschluss, wenn der Führungsdraht entfernt wurde, versehen ist.

8. Vorrichtung nach Anspruch 7, wobei das Verschlusselement ein Rückschlagventil 36 ist.

9. Vorrichtung nach einem vorangehenden Anspruch, wobei der Führungsdrahtkanal auch als Spülkanal für die Einbringung steriler Spüllösung in die Blase, nachdem der Führungsdraht entfernt wurde, fungiert.

10. Vorrichtung nach einem vorangehenden Anspruch, wobei der Führungsdraht ein hydrophiles Polymermaterial umfasst.

11. Vorrichtung nach einem vorangehenden Anspruch, wobei die distale Spitze des Katheters für die Ableitung von Harn aus der Blase offenendig ist und schräg ausgebildet ist.

12. Vorrichtung nach einem vorangehenden Anspruch, wobei der Katheter ferner einen Aufblaskanal zum Einbringen von Aufblasfluid in einen neben der distalen Spitze des Katheters befindlichen aufblasbaren Ballon zum Halten des Katheters im Inneren der Blase umfasst.

13. Harnkatheterisierungs-Kit, Folgendes umfassend:
einen Katheter 10 mit einem länglichen Körper 12, der mit einer distalen Spitze 16, einem proximalen Ende 18 und einem Ableitungskanal 14 und einem separaten Führungsdrahtkanal 32, die sich dadurch erstrecken, versehen ist, wobei der Ableitungskanal sich entlang der Mitte des länglichen Körpers erstreckt und der Führungsdrahtkanal sich parallel zu dem Ableitungskanal entlang einer Seite des länglichen Körpers erstreckt;
einen Führungsdraht 38, der sich durch den Führungsdrahtkanal erstreckt, zum Führen des Katheters beim Einlegen;
einen Gleitmittelzufuhranschluss 34 an oder nahe dem proximalen Ende des länglichen Körpers, wobei der Gleitmittelzufuhranschluss einen in Fluidverbindung mit dem Führungsdrahtkanal stehenden Seitenarm für die Einbringung eines Führungsdraht-Gleitfluids in den Führungsdrahtkanal umfasst; und
eine sterile Verpackung 90, in welcher der Katheter und der Führungsdraht zum Gebrauch bereitgestellt werden, wobei die Verpackung dazu angepasst ist, geöffnet zu werden, um die Injektion von Führungsdraht-Gleitfluid über den Gleitmittelzufuhranschluss zu gestatten, um den Führungsdraht in dem Führungsdrahtkanal und denjenigen Abschnitt des Führungsdrahtes, der sich über die distale Spitze des Katheters hinaus erstreckt, mit Gleitmittel zu versehen.

14. Kit nach Anspruch 13, der ferner eine äußere sterile Verpackung 104 zum Aufnehmen der sterilen Verpackung, die den Katheter und den Führungsdraht enthält, umfasst, wobei die äußere sterile Verpackung zusätzlich mindestens eine Spritze 108 aufnimmt und wobei die mindestens eine Spritze mit Führungsdraht-Gleitfluid vorgefüllt ist.

## Revendications

1. Dispositif de cathétérisation urinaire comprenant :
un cathéter 10 ayant un corps allongé 12 muni d'une extrémité distale 16, d'une extrémité proximale 18, et d'un canal de drainage 14 et d'un canal de fil-guide séparé 32 s'étendant à travers celui-ci ;
un fil-guide 38 s'étendant à travers le canal de fil-guide permettant de guider le cathéter lors de l'insertion ; et
un orifice de lubrification 34 au niveau ou vers l'extrémité proximale du corps allongé, l'orifice de lubrification comprenant un bras latéral en communication fluidique avec le canal de fil-guide pour l'introduction d'un fluide de lubrification du fil-guide dans le canal de fil-guide ;
le canal de drainage s'étendant le long du centre du corps allongé et le canal de fil-guide s'étendant parallèlement au canal de drainage le long d'un côté du corps allongé.

2. Dispositif selon la revendication 1, dans lequel le fil-guide est muni d'un obturateur 50 permettant d'empêcher l'extrémité proximale du fil-guide de passer à travers le canal de fil-guide vers l'extrémité distale du cathéter.

3. Dispositif selon la revendication 2, dans lequel l'obturateur est adapté pour recevoir une seringue permettant d'introduire le fluide de lubrification du fil-guide dans le canal de fil-guide lorsque l'obturateur est connecté à l'orifice de lubrification.

4. Dispositif selon la revendication 2 ou la revendication 3, dans lequel l'obturateur peut être raccordé de manière amovible à l'orifice de lubrification.

5. Dispositif selon l'une quelconque des revendications 2 à 4, dans lequel l'obturateur est muni d'une cheville 58 permettant de raccorder l'extrémité proximale du fil-guide à l'obturateur, la cheville étant conçue de manière à permettre au fluide de lubrification du fil-guide de s'écouler autour de la cheville lors de son passage à travers l'obturateur et dans le canal de fil-guide.

6. Dispositif selon la revendication 5, dans lequel la cheville comprend une bande qui traverse la largeur d'un noyau creux de l'obturateur, et des éléments d'extrémité au niveau de chaque extrémité de la bande, qui sont intégrés de chaque côté du bouchon afin de fixer la cheville dans l'obturateur.

7. Dispositif selon l'une quelconque des revendications précédentes, dans lequel l'orifice de lubrification est muni d'un élément de fermeture permettant d'empêcher le drainage de l'urine par l'orifice de lubrification lorsque le fil-guide a été enlevé.

8. Dispositif selon la revendication 7, dans lequel l'élément de fermeture est un clapet anti-retour 36.

9. Dispositif selon l'une quelconque des revendications précédentes, dans lequel le canal de fil-guide sert également de canal d'irrigation pour l'introduction d'une solution d'irrigation stérile dans la vessie une fois que le fil-guide a été retiré.

10. Dispositif selon l'une quelconque des revendications précédentes, dans lequel le fil-guide comprend un matériau polymère hydrophile.

11. Dispositif selon l'une quelconque des revendications précédentes, dans lequel l'extrémité distale du cathéter est ouverte pour permettre le drainage de l'urine provenant de la vessie et est formée selon un angle oblique.

12. Dispositif selon l'une quelconque des revendications précédentes, dans lequel le cathéter comprend en outre un canal de gonflage permettant d'introduire un fluide de gonflage dans un ballonnet gonflable adjacent à l'extrémité distale du cathéter afin de retenir le cathéter dans la vessie.

13. Kit de cathétérisation urinaire comprenant :
un cathéter 10 ayant un corps allongé 12 muni d'une extrémité distale 16, d'une extrémité proximale 18, et d'un canal de drainage 14 et d'un canal de fil-guide séparé 32 s'étendant à travers celui-ci, le canal de drainage s'étendant le long du centre du corps allongé et le canal de fil-guide s'étendant parallèlement au canal de drainage suivant un côté du corps allongé ;
un fil-guide 38 s'étendant à travers le canal de fil-guide afin de guider le cathéter lors de son insertion ;
un orifice de lubrification 34 au niveau ou vers l'extrémité proximale du corps allongé, l'orifice de lubrification comprenant un bras latéral en communication fluidique avec le canal de fil-guide permettant l'introduction d'un fluide de lubrification du fil-guide dans le canal de fil-guide ; et
un emballage stérile 90 dans lequel le cathéter et le fil-guide sont fournis pour une utilisation, l'emballage étant adapté pour être ouvert afin de permettre l'injection de fluide de lubrification du fil-guide à travers l'orifice de lubrification afin de lubrifier le fil-guide dans le canal de fil-guide et cette partie du fil-guide s'étendant au-delà de l'extrémité distale du cathéter.

14. Kit selon la revendication 13 comprenant en outre un emballage stérile externe 104 permettant de loger l'emballage stérile contenant le cathéter et le fil-guide, l'emballage stérile externe contenant en outre au moins une seringue 108, et la au moins une seringue étant préremplie de fluide lubrifiant pour fil-guide.
